# EUROPEAN PATENT SPECIFICATION

(11) **EP 2 685 936 B1**
(45) Date of publication and mention of the grant of the patent: **11.05.2016**
(21) Application number: 11709926.7
(22) Date of filing: 18.03.2011
(51) Int. Cl.: A61F 2/38

(54) **A TIBIAL TRAY FOR A KNEE JOINT PROSTHESIS AND A KNEE JOINT PROSTHESIS INCLUDING SAME**
TIBIAPLATEAU FÜR EINE KNIEGELENKPROTHESE UND KNIEGELENKPROTHESE DAMIT
PLATEAU TIBIAL POUR PROTHÈSE DU GENOU ET PROTHÈSE DU GENOU LE COMPRENANT

(43) Date of publication of application: 22.01.2014
(73) Proprietor: Smith & Nephew Orthopaedics AG, 6340 Baar (CH)
(72) Inventor: HOCHULI, Patrick, 5057 Reitnau (CH)
(74) Representative: Smith & Nephew
(86) International application number: PCT/EP2011/054139
(87) International publication number: WO 2012/126496

(56) References cited:
- EP-A2- 1 872 746
- DE-A1-102004 056 713
- US-A- 4 963 153
- US-A1- 2003 060 884
- US-A1- 2006 235 537
- US-A1- 2010 249 941

## Description

The present invention relates to a tibial tray for a knee joint prosthesis and to a knee joint prosthesis including such a tibial tray. The closest prior art is document US 2003/0060884 A1, which defines the preamble of claim 1.

Many varying prosthetic devices are available for knee joint replacement. These include bicondylar prosthetic devices, where both condyles of the knee are replaced, and unicondylar prosthetic devices wherein only of the knee condyles is replaced. In such a procedure, tibial components are used to replace either or both of the medial and lateral condyles of the patient. A single, total femoral component or two partial femoral components may then be used to cooperate with the tibial component or components. However, unicondylar prostheses have to be precisely installed otherwise they are prone to failure. The Oxford unicondylar knee system was introduced to overcome this problem. In this system, a floating insert is located between a tibial component in the form of a tibial tray and the femoral component so that the insert can move with respect to both the tibial tray and the femoral component. This reduces the degree of precision required to produce a satisfactory knee joint but it is still necessary for the tibial and femoral components to be installed carefully.

One aim of the present invention is to provide a tibial tray for use in such a unicondylar prosthetic device that facilitates its positioning during surgery in the tibia of a patient and that therefore increases the degree of precision that can be achieved during its installation.

A further aim is to provide a knee joint prosthesis including the aforementioned tibial tray that includes a floating insert adapted for use with the tibial tray.

According to a first aspect of the present invention there is provided a tibial tray for a knee joint prosthesis as defined claim 1.

Advantageously, an apex of the triangular keel is between 6 mm and 11mm from the distal fixation surface.

Preferably also, an angle between the distal edge of the keel and the proximal bearing surface is between 10° and 30° inclusive and is preferably approximately 17°.

Preferably also, the keel extends in a sagittal plane over half the length of the anterior-posterior length of the tray.

Preferably also, the proximal bearing surface is smooth and generally planar.

Preferably also, the distal fixation surface of the platform is configured for attachment to a tibia by a bone cement.

Preferably also, the distal fixation surface of the platform is provided with one or a plurality of cement pockets. Advantageously, each of the cement pockets is aligned in an anterior-posterior direction.

Preferably also, the distal fixation surface of the platform is roughened.

Preferably also, the platform defines a wall that projects in a proximal direction at an intercondylar edge of the proximal bearing surface. Advantageously, the wall extends proximally, in use, in a sagittal plane.

According to a second aspect of the present invention there is provided a knee joint prosthesis comprising a tibial tray according to the first aspect of the present invention.

Preferably, the knee joint prosthesis additionally comprises a femoral component adapted for attachment to a femur, and a floating insert adapted to fit between the femoral component and the tibial tray when the prosthesis is implanted in a patient, the insert having a femoral side shaped to interface slidingly with an outer surface of the femoral component during flexion of the knee and a tibial side shaped to interface slidingly with the proximal bearing surface of the tibial tray.

Preferably also, the insert is not symmetric about a sagittal plane. Advantageously, the insert has an intercondylar side that is longer in length than an opposite superficial side.

Preferably also, a corner at a posterior end of the intercondylar side of the insert is extended to project rearwardly.

Preferably also, an angle between the intercondylar side and a posterior side of the insert is less than 90°.

Preferably also, corners between the superficial side of the insert and its posterior and anterior sides arc rounded.

Preferably also, a surface of the tibial side of the insert is smooth and generally planar.

Preferably also, an anterior-posterior profile of the insert is substantially wedge-shaped with the anterior height greater than the posterior height.

Preferably also, in the sagittal plane the difference in height between a deepest point of the femoral side of the insert and a posterior edge of the femoral side of the insert is no greater than 3 mm and is preferably of the order of 2mm.

Preferably also, in the sagittal plane the difference in height between a deepest point of the femoral side of the insert and an anterior edge of the femoral side of the insert is between 2mm and 6 mm inclusive and is preferably of the order of 4 mm.

Preferably also, said outer surface of the femoral component defines two regions for flexion rotation in a sagittal plane of which a first region has a first radius of curvature and a second region has a second radius of curvature, the first radius of curvature being larger than the second radius of curvature, and in that the surface of the femoral side of the insert has substantially the same radius of curvature in the sagittal plane as the first radius of curvature of the femoral component.

Preferably also, said outer surface of the femoral component has a third radius of curvature for varus/valgus rotation in a coronal plane, and in that the surface of the femoral side of the insert has substantially the same radius of curvature in the coronal plane as the third radius of curvature of the femoral component.

Preferably also, the knee joint prosthesis comprises a unicondylar prosthetic device.

The various aspects of the present invention will now be described by way of example with reference to the accompanying drawings, in which:-
- Fig. 1: is a side view of a knee joint prosthesis including a tibial tray, a femoral component and a floating insert;
- Figs. 2 to 4: are respectively top, bottom anterior elevations of an embodiment of tibial tray forming part of the prosthesis similar to that shown in Fig. 1 and in accordance with the present invention;
- Fig. 5: is a cross-section along the line V-V in Fig. 4;
- Fig. 6: is a cross-section along the line VI-VI in Fig. 3;
- Figs. 7 and 8: are perspective views of the tray from above and below respectively.
- Fig. 9: is a perspective view of a floating insert forming part of the knee joint prosthesis shown in Fig. 1;
- Figs. 10 and 11: are respectively plan and anterior views of the insert shown in Fig. 9;
- Fig. 12: is a cross-section along the line XII-XII in Fig. 10;
- Fig. 13: is a cross-section along the line XIII-XIII in Fig. 10;
- Figs 14 and 15: are side views of the knee joint prosthesis similar to that shown in Fig. 1 showing the position of the floating insert relative to the tibial tray and the femoral component when the femoral component is positioned at 8° hyper-extension and at 125° flexion respectively;
- Figs. 16 to 18: are side views of the knee joint prosthesis similar to that shown in Fig. 1 but with the floating insert shown in cross-section along a sagittal place and with a femoral component of the prosthesis positioned at 0°, 30° and 80° of flexion respectively; and
- Fig. 19: is a cross-section along the line IXX-IXX of Fig. 17.

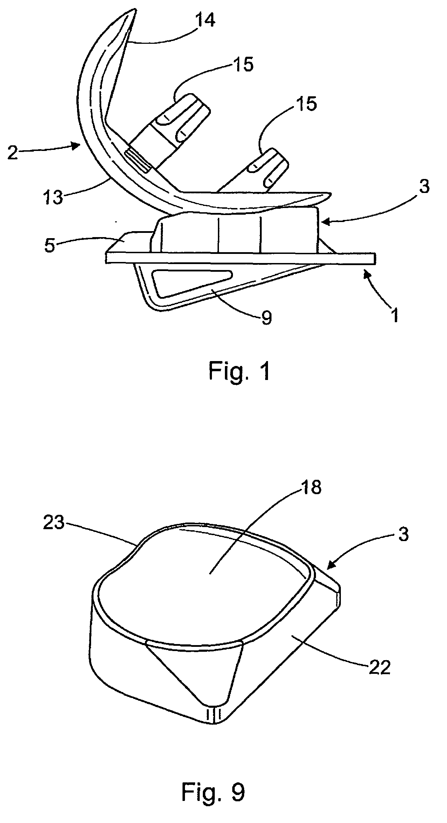

A unicondylar knee joint prosthesis such as is shown in Fig. 1 comprises a tibial tray 1, a femoral component 2 and a floating insert 3. The advantage of such prostheses is that they can be implanted with minimal invasive surgical procedures and involve a small amount of bone resection. Both the tibial tray 1 and the femoral component 2 are preferably made of a material that provides a smooth sliding surface, is sufficiently rigid and durable, and will not cause adverse patient reactions, for example a cobalt chromium molybdenum alloy or oxidized zirconium. In contrast, the floating insert 3 is preferably made of a material that can provide a good sliding interface with both the tibial tray 1 and the femoral component 2 and that also provides good friction and wear characteristics. An example of a suitable material for the insert 3 is ultra high molecular weight polyethylene, a ceramic, oxidized zirconium or a combination of these. The tibial tray 1 and the femoral component 2 are both designed to be cemented in position. Conventional bone cements, such as those based on polymethylmethacrylate can be used.

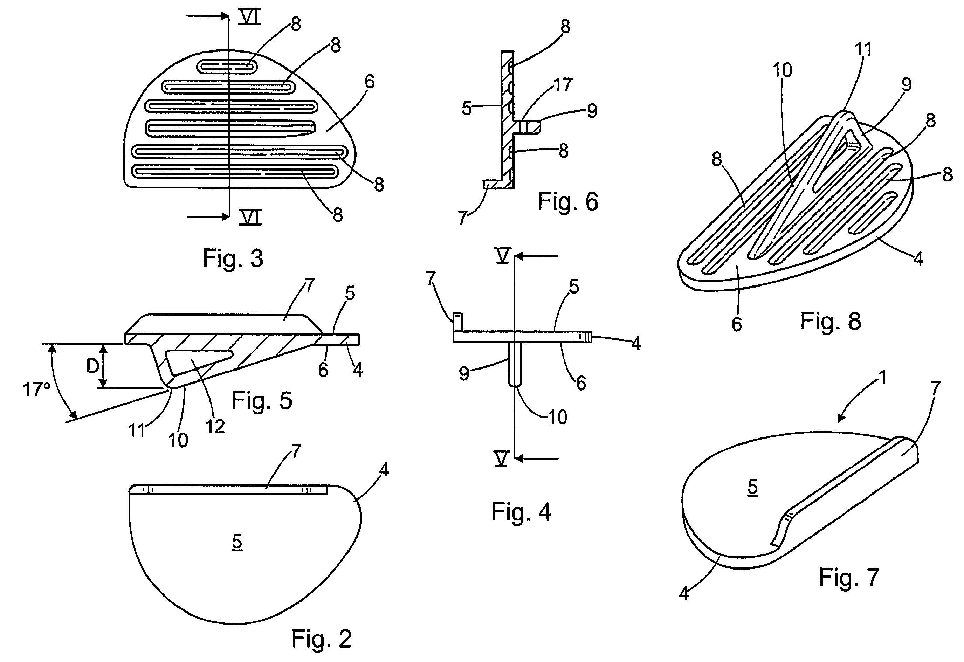

An embodiment of the tibial tray 1 for use in such a prosthesis is shown in Figs. 2 to 8. It should be appreciated that the embodiment of tray 1 shown in Figs. 1 and 14 to 19 of the drawings is shaped for implantation in the lateral side of a left knee or the medial side of a right knee of a patient. A tray suitable for implantation in the medial side of a left knee or the lateral side of a right knee of a patient would be a mirror image of this tray and is as shown in Figs. 2 to 8 of the drawings.

The tibial tray 1 comprises a platform 4 with a proximal bearing surface 5 and a distal fixation surface 6. The platform 4 is typically 2mm thick but may be between 1.5 mm and 3 mm thick dependent on the overall size of the tray and any particular patient requirements. The proximal bearing surface 5 is smooth and generally planar. At the intercondylar edge of the platform 4 is a projecting wall 7 that is linear and that in use extends in a sagittal plane from the proximal bearing surface 5 and projects in a proximal direction. The wall 7 is provided to prevent dislocation of the insert 3 during use and to prevent abrasion of the insert 3 by adjacent bone. In contrast, the superficial, anterior and posterior edges 5 of the platform 2 form a continuous, asymmetric curve which approximates to the exterior profile of the underlying bone so that there is minimal impairment of the function of the cruciate ligaments and leg muscles when the implant is in use.

The distal fixation surface 6 is configured for attachment to a tibia and is preferably roughened, for example by sand-blasting to provide a key for cementation or by vacuum plasma spray (VPS), for example, for a cementless version of the implant. In addition, the distal fixation surface 6 is provided with one or a plurality of cement pockets 8 that are preferably less than 1.0 mm deep and typically around 0. 5 mm deep. These pockets 8 are aligned parallel to one another and are preferably in an anterior-posterior direction as if they extended in a medial-lateral direction, the platform 4 may be weakened too much. Although with a thicker platform 4, such an alignment is possible.

Projecting from the distal fixation surface 6 is a keel 9 that extends in an anterior-posterior direction with respect to the position of the tray 1 when in use. The keel 9 therefore extends in a direction parallel to the cement pockets 8 and is located approximately centrally of the tibial platform 4 in a medial-lateral direction. This is important as it increases the strength of the tray 1. The keel 9 is substantially planar in a sagittal plane and triangular in shape with a distal edge 10 that extends obliquely from an apex 11 with respect to the proximal bearing surface 5 of the platform 4. Preferably, the apex 11, which is at the deepest part of the keel 9, has a depth D that is between 3 mm and 15 mm from the distal fixation surface 6. Preferably also, the angle between the distal edge 10 of the keel 9 and the proximal bearing surface 5 is between 10° and 30° inclusive and is preferably around 17° with a few degrees difference either way. The deepest part of the keel 9 is also towards the posterior aspect of the tray 1. Overall, the keel 9 extends in a sagittal plane along the greater part of the anterior-posterior length of the platform 4 and therefore the tray 1. Typically, the anterior end of the distal edge 10 is approximately 6.2 mm from anterior edge of the platform 4 and the posterior end of the distal edge 10 is approximately 5.0 mm from the posterior edge of the platform 4. As the overall anterior-posterior length of the platform 4 may be between approximately 38.0 mm and 55.4 mm dependent on the tibial plateau size of the patient it will be appreciated that in all cases the keel 9 extends over half the length of the tray 1. To ensure a strong bond between the tray 1 and the tibia, the central section of the keel 9 may be pierced by an aperture 12 to allow cement to flow through it during implantation. The shape of the aperture 12 may mirror the triangular shape of the keel 11.

The keel 9 facilitates positioning and fixation of the tray 1 during implantation to a resected end of a tibia. The surgical method of implanting the tibial tray 1 involves resecting at least a portion of a condyle of a patient's tibia to create a surgically-prepared tibial surface. The surgeon may then form a slot in the surgically-prepared tibial surface that extends posteriorly from an opening formed in an anterior surface of the tibia. When positioning the tibial tray 1, the keel 9 enables the tray 1 to be slid into place over the top of the prepared tibial surface from anterior to posterior into the tibial resection and enables the platform 4 of the tray 1 to be located with precision over the resected end of the tibia. Bone cement may be used to secure the tray 1 to the resected tibia and may be injected into the slot once the tray 1 has been correctly positioned.

As indicated above, the tibial tray 1 forms part of a unicondylar knee joint prosthesis as shown in Fig. 1. The femoral component 2 of this prosthesis has a curved smooth outer surface 13 that forms a sliding interface with the insert 3, as is described in more detail below. Opposed to the outer surface 13 is a fixation surface 14, which may be roughened, for example by sand-blasting or by vacuum plasma spray (VPS), from which two aligned pegs 15 project to enable the component 2 to be positioned and secured to a femur so that the component 2 is prevent from rotating relative to the femur.

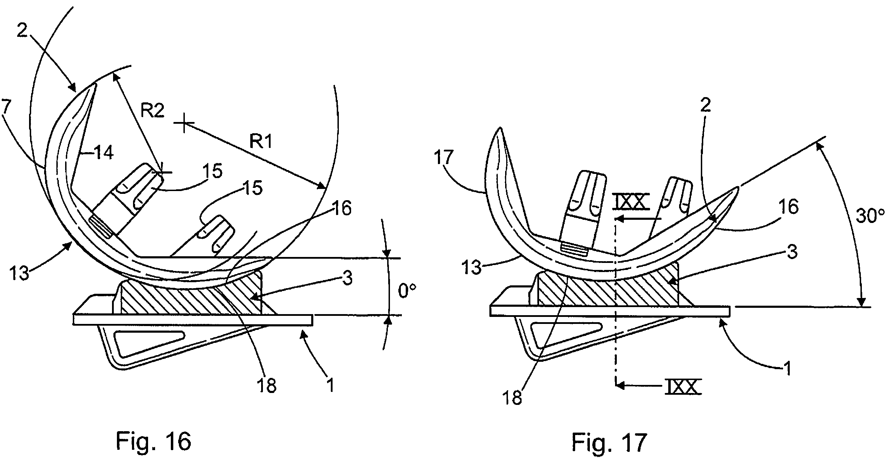

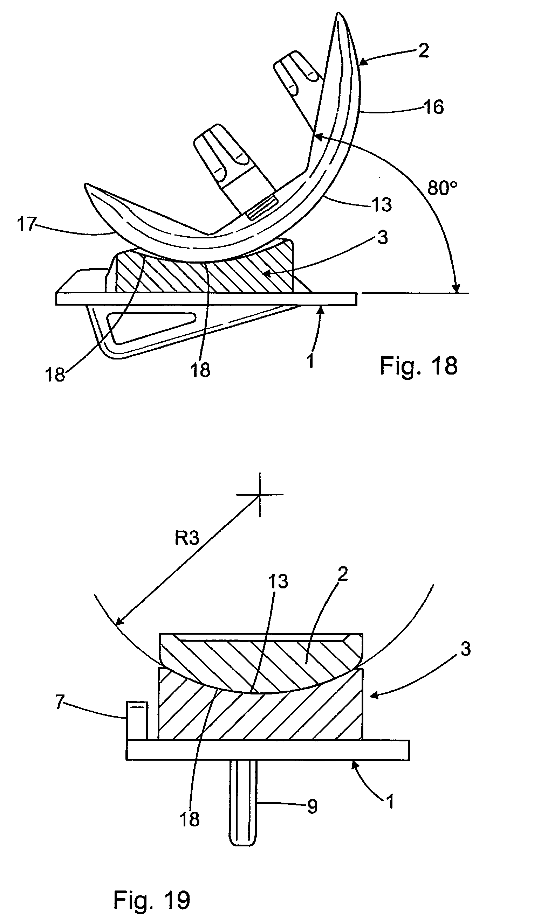

As shown in Figs. 16 to 19, in the sagittal plane, the surface 13 of the femoral component 2 defines two regions 16 and 17 for flexion rotation in a sagittal plane of which the first, r region 16 has a first radius of curvature R1 and the second region 17 has a second radius of curvature R2. The surface 17 with the second radius of curvature R2 defines the posterior part of the component 2 while the surface 16 with the first radius of curvature R1 defines the anterior part of the component 2 and imitates the distal shape of the femoral condyles. The first radius of curvature R1 is larger than the second radius of curvature R2 and typically the ratio of these two radii is between 1.3:1 and 1.8:1. The surface 13 of the femoral component 2 also has a third radius of curvature R3 in a coronal plane for varus/valgus rotation, as shown in Fig. 19. The radius of curvature R3 is substantially the same as the radius of curvature R2. This means that die surface of the posterior part of the component 2 is spheroidal whereas the surface of the distal or anterior part of the component 2 is toroidal.

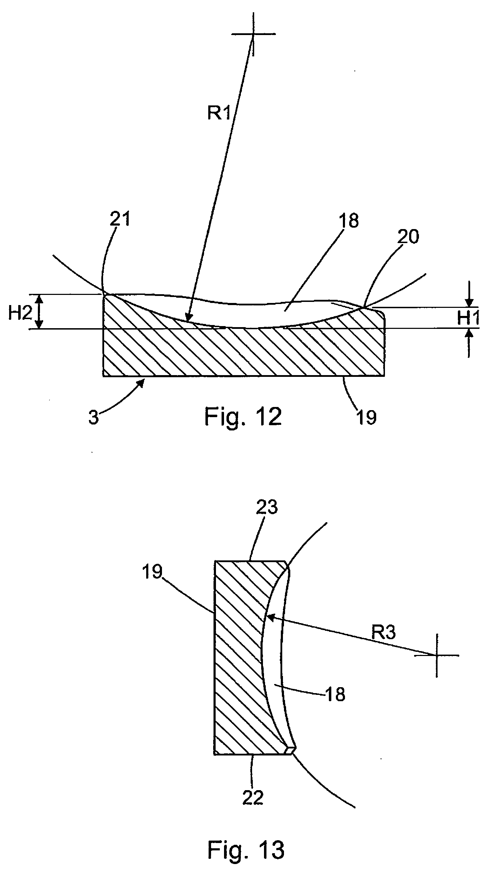

The floating insert 3 is designed to slide between the proximal bearing surface 5 of the tibial tray 1 and the outer surface 13 of the femoral component 2. An embodiment of it will now be described in more detail with reference to Figs. 9 to 13.

The insert 3 has a concave femoral side 18 shaped to interface slidingly with the surface 13 of the femoral component 2 during flexion of the knee and a tibial side 19 shaped to interface slidingly with the proximal bearing surface 5 of the tibial tray 1. The surface of the tibial side 19 of the insert 3 is therefore smooth and generally planar like the proximal bearing surface 5 of the tibial tray 1. In addition, the anterior-posterior profile of the insert 3 is substantially wedge-shaped with the anterior height greater than the posterior height so that it can be pushed into position between the tibial tray 1 and the femoral component 2. The difference in height between the lowest and highest parts of the concave femoral side 18 defines the resistance against luxation of the prosthesis. However, in the sagittal plane the difference H1 in height between the deepest point of the femoral side 18 and a posterior edge 20 of the femoral side 18 should be no greater than around 3 mm and is preferably of the order of 2 mm otherwise too much force is required to push the insert 3 into position between the tibial tray 1 and the femoral component 2. Luxation towards the anterior is prevented by a difference in height H2 between the deepest point of the femoral side of the insert 18 and an anterior edge 21 of the femoral side 18 is between 2mm and 6 mm inclusive and is preferably of the order of 4 mm.

The concave nature of the femoral side 18 of the insert 3 is such that its surface has substantially the same radius of curvature in the sagittal plane as the first radius of curvature R1 of anterior part of the femoral component 2. This means that there is maximum congruency between the insert 3 and the femoral component 2 in extension. In addition, this surface in the coronal plane has substantially the same radius of curvature as the third radius of curvature R3 of the femoral component 2. This allows varus/valgus rotation of the knee in a coronal plane of up to 8°.

Although it is possible for the insert 3 to be symmetric about a sagittal plane, preferably it is asymmetric as shown in Figs. 9 to 13. It should be noted in this regard that the embodiment shown in Figs. 9 to 13 is shaped for implantation in the medial side of a left knee or the lateral side of a right knee of a patient and therefore for use with a tibial tray 1 as shown in Figs. 2 to 8. An insert suitable for implantation in the lateral side of a left knee or the medial side of a right knee of a patient would be a mirror image of the insert 3 illustrated in these drawings.

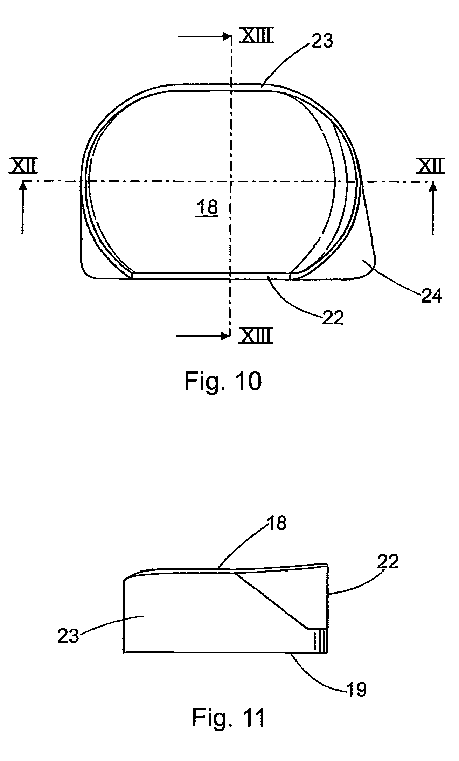

As shown, overall the insert 3 is not symmetric about its sagittal plane although the surface defined by the femoral side 18 is symmetric about this plane and is approximately rectangular but with rounded corners. Beneath this femoral surface, the insert 3 is shaped to prevent excess rotation relative to the tibial tray 1 from taking place during use and to protect surrounding soft tissues from damage. It has an intercondylar side 22 that extends linearly and that is adapted for location adjacent the wall 7 of the tibial tray 1. This side 22 is longer in length than an opposite superficial side 23 of the insert 3, which has a rounded profile. Contact between the longer intercondylar side 22 of the insert 3 and the wall 7 limits extensive rotation of the insert 3 relative to the tibial tray 1 as the knee joint is moved. In particular, the corner 24 at the posterior end of the intercondylar side 22 may be extended and project rearwardly as shown in Fig. 10 for the express purpose of preventing extensive rotation relating to the tibial tray 1. The angle A1 between the intercondylar side 22 and the posterior side of insert 3 is therefore less than 90° and is typically around 80°. The corner A2 between the intercondylar side 22 and the anterior side of the insert is equal to or less than 90° although the actual apexes of both corners are rounded. However, the corners between the superficial side 15 of the insert and its posterior and anterior sides are rounded in order to protects the surrounding soft tissue from damage during deep flexion and during hyperextension of the knee.

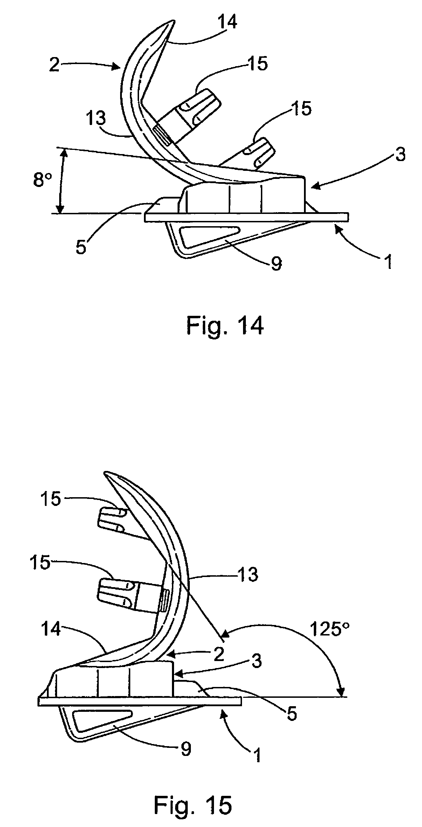

The generally planar nature of the proximal bearing surface 5 of the tibial tray 1 means that it is not able to guide motion between the femur and the tibia of the patient during movement of the knee joint. This has to be controlled by the patient's own muscles, the capsule and the ligaments around the knee joint. It is therefore important that the prosthesis is implanted with precision in a manner which causes minimum damage to the soft tissues around the knee. The prosthesis is designed to allow a range of motion between 8° hyper-extension to 125° flexion. These two positions are shown in Figs. 14 and 15 respectively. In hyper-extension, when the tibia is angled forwardly from the knee joint, the insert 3 sits approximately centrally in an anterior-posterior direction on the tibial tray 1, as shown in Fig. 14. However, during flexion, the femoral component 1 bears on the insert 3 and slides it along the proximal bearing surface 5 towards the posterior of the tray 1. At approximately 125° of flexion, which is typically maximum flexion, the posterior edge 20 and side of the insert 3 is very close to the posterior edge of the proximal bearing surface 5 of the tray 1, as shown in Fig. 15.

Flexion beyond 125° depends on the way any bony overhang on the posterior femoral condyle is treated. Such an overhang occurs mainly when the sizing or placement of the implant is not accurate and preferably any overhang is removed by a chipping it off with a curved chisel to prevent impingement between the femur and the insert 3 at greater than 125° of flexion.

During flexion, congruence between the insert 3 and the tibial tray 1 is presumed as the proximal bearing surface 5 and the tibial side 19 of the insert 3 are both generally planar. However, the contact condition between the surface 13 of the femoral component 2 and the femorale side 18 of the insert 3 varies with the degree of flexion. In full extension at 0° of flexion, as shown in Fig. 16, the sagittal and frontal radii are the same so that the surfaces 13 and 18 are totally congruent. This situation is maintained until approximately 30° of flexion when the region 17 witch the smaller posterior radius R2 of the surface component 2 comes into contact with the surface 18, as shown in Fig. 17. Between approximately 30° and 80° of flexion, the latter of which is shown in Fig. 18, both regions 16 and 17 of the surface 13 are substantially in contact with the surface 18 of the insert 3 and the contact area between the component 2 and the insert 3 is reduced. Beyond approximately 80° only the region 17 of the surface 13 is in contact with the surface 18 of the insert 3 and the contact area between the component 2 and the insert 3 is at its smallest. However, the load between them is at its smallest too. The largest loading between the two components 2 and 3 occurs between 0° and 30° of flexion when they are both full congruent.

Implantation of the prosthesis is carried out in accordance with the following steps. It should be noted that the size of the tibial tray 1 and the femoral component 2 to be selected for a patient should be determined pre-operatively but should be verified during surgery using trial parts and templates. It will be appreciated that the femoral component 2 will replace either the medial or lateral femoral condyle being removed while the tibial tray 1 defines the plane for the anterior-posterior and medial-lateral movement direction of the floating insert dependent on the tibial resection plane.

During surgery, the tibia of the patient is prepared first. The orientation of the tibial resection is adjusted by an extramedullary guide. Its distal end is placed around the patient's ankle and then it is aligned to the tibial axis. The proximal end of the extramedullary guide is fixed to the tibia using the guide's built-in spikes or with pins through the cutting block. A tibial cutting block is also used and if no posterior slop is desired, the sagittal plane is adjusted by using the extramedullary guide. The resection depth is then determined using a stylus by touching the articular surface of the tibial plateau. The sagittal cut should be resected just medial to the ACL attachment point on the tibial spine in order to maximize the size of the tibial base. The tibial tray 1 is fitted to the resected tibia and the keel 9 supports positioning and fixation of the platform 4 to the tibia. The oblique shape of the keel 9 facilitates the cutting of a groove in the resected tibia to enable the sliding of the tray 1 into position from anterior to posterior into the tibial resection. The position of the keel 9 is positioned roughly in the centre of the tibial tray 1 and therefore is roughly central beneath the insert 3 in order to increase the strength of implant. The keel 9 also anchors the tibial tray 1 into the tibial bone.

The tibial resection is used to measure the joint gap in both flexion and extension, the extension gap being measured at 20° flexion. The difference between the extension gap and the flexion gap relates to possible cartilage damage or ligament disbalance and should be adjusted using spacers which are assembled with the distal femoral resection guide. These correct the distal femoral cut so that there is a constant joint gap throughout the whole range of motion. The size of the insert 3 is chosen according to the size of this flexion gap.

Resection of the distal femur is performed in full extension using the same cutting block and an appropriate spacer. The posterior and the chamfer cut are resected using a second cutting block, which also guides the drilling of the holes for the posts 15. The femoral component 2 is fitted to the resected femur using the posts 15, which are preferably aligned under 30° to the posterior cut and are also perpendicular to the posterior-distal chamfer. Thus facilitates the intra-operative placement of the component 2 and prevents its loosening in deep flexion. The femoral component 2 is then cemented into position to the resected distal femur. After fitment of both the tibial tray 1 and the femoral component 2, the insert 3 is pushed into position between them.

### Reference Numerals

- 1: Tibial tray
- 2: Femoral component
- 3: Floating insert
- 4: Platform
- 5: Proximal bearing surface
- 6: Distal fixation surface
- 7: Intercondylar wall
- 8: Cement pocket
- 9: Keel
- 10: Distal edge of keel
- 11: Apex of keel
- 12: Aperture through keel
- 13: Outer surface of femoral component
- 14: Fixation surface of femoral component
- 15: Posts
- 16: Anterior part of outer surface
- 17: Posterior part of outer surface
- 18: Femoral side of insert
- 19: Tibial side of insert
- 20: Posterior edge
- 21: Anterior edge
- 22: Intercondylar side
- 23: Superficial side
- 24: Posterior intercondylar corner

- A1: Angle between posterior and intercondylar sides of insert
- A2: Angle between anterior and intercondylar sides of insert
- D: Depth of keel
- R1: Radius of anterior part of femoral component
- R2: Radius of posterior part of femoral component
- R3: Radius in coronal plane
- H1: Posterior height of insert
- H2: Anterior height of insert

## Claims

1. A tibial tray (1) for a knee joint prosthesis, comprising
a platform (4) including a proximal bearing surface (5) and a distal fixation surface (6); and
a keel (9) that projects from the distal fixation surface (6) and that extends in an anterior-posterior direction with respect to the position of the tray (1) when in use; wherein the keel (9) is located centrally of the tibial tray (1) in a medial-lateral direction and is substantially planar in a sagittal plane with a distal edge (10) that extends obliquely with respect to the proximal bearing surface (5) of the platform (4); wherein the keel (9) is substantially triangular in shape to define a distal edge (10) having an apex (11) , wherein the apex (11) is located towards a posterior aspect of the tray (1) and **characterized in that** the anterior and posterior ends of the distal edge (10) are located on the distal fixation surface (6) and spaced apart from respective edges of the platform (4).

2. A tibial tray (1) as claimed in Claim 1, **characterised in that** the apex (11) of the triangular keel (9) is between 6 mm and 11 mm from the distal fixation surface (6).

3. A tibial tray (1) as claimed in Claims 1 or 2, **characterised in that** an angle between the distal edge (10) of the keel (9) and the proximal bearing surface (5) is between 10° and 30° inclusive and is preferably approximately 17°.

4. A tibial tray (1) as claimed in any preceding Claim, **characterised in that** the keel (9) extends in a sagittal plane over half the length of the anterior-posterior length of the tray (1).

5. A tibial tray (1) as claimed in any preceding Claim, **characterised in that** the proximal bearing surface (5) is smooth and generally planar.

6. A tibial tray (1) as claimed in any preceding Claim, **characterised in that** the distal fixation surface (6) of the platform (4) is configured for attachment to a tibia by a bone cement.

7. A tibial tray (1) as claimed in any preceding Claim, **characterised in that** the distal fixation surface (6) of the platform is provided with one or a plurality of cement pockets (8).

8. A tibial tray (1) as claimed in Claim 7, **characterised in that** each of the cement pockets (8) is aligned in an anterior-posterior direction.

9. A tibial tray (1) as claimed in any preceding Claim, **characterised in that** the distal fixation surface (6) of the platform is roughened.

10. A tibial tray (1) as claimed in any preceding Claim, **characterised in that** the platform (4) defines a wall (7) that projects in a proximal direction at an intercondylar edge of the proximal bearing surface (5).

11. A tibial tray (1) as claimed in Claim 10, **characterised in that** the wall (7) extends proximally, in use, in a sagittal plane.

12. A knee joint prosthesis comprising a tibial tray (1) as claimed in any preceding Claim.

13. A knee joint prosthesis as claimed in Claim 12 , that additionally comprises:
a femoral component (2) adapted for attachment to a femur, and
a floating insert (3) adapted to fit between the femoral component (2) and the tibial tray (1) when the prosthesis is implanted in a patient, the insert (3) having a femoral side (18) shaped to interface slidingly with an outer surface (13) of the femoral component (2) during flexion of the knee and a tibial side (19) shaped to interface slidingly with the proximal bearing surface (5) of the tibial tray (1),

14. A knee joint prosthesis as claimed in Claim 13, **characterised in that** the insert (3) is not symmetric about a sagittal plane.

15. A knee joint prosthesis as claimed in Claim 14, **characterised in that** the insert (3) has a intercondylar side (22) that is longer in length than an opposite superficial side (23).

16. A knee joint prosthesis as claimed in Claim 14 or 15, **characterised in that** a corner (24) at a posterior end of the intercondylar side (22) of the insert (3) is extended to project rearwardly,

17. A knee joint prosthesis as claimed in any of Claims 14 to 16, **characterised in that** an angle (A1) between the intercondylar side (22) and a posterior side of the insert (3) is less than 90°.

18. A knee joint prosthesis as claimed in any of Claims 13 to 17, **characterised in that** corners between a superficial side (23) of the insert (3) and its posterior and anterior sides are rounded.

19. A knee joint prosthesis as claimed in any of Claims 13 to 18, **characterised in that** a surface of the tibial side (1) of the insert (3) is smooth and generally planar.

20. A knee joint prosthesis as claimed in any of Claims 13 to 19, **characterised in that** an anterior-posterior profile of the insert (3) is substantially wedge-shaped with the anterior height (H2) greater than the posterior height (H1).

21. A knee joint prosthesis as claimed in any of Claims 13 to 20, **characterised in that** in the sagittal plane the difference in height between a deepest point of the femoral side (18) of the insert (3) and a posterior edge (20) of the femoral side (18) of the insert (3) is no greater than 3 mm and is preferably of the order of 2mm.

22. A knee joint prosthesis as claimed in any of Claims 13 to 21, **characterised in that** in the sagittal plane the difference in height between a deepest point of the femoral side (18) of the insert (3) and an anterior edge (21) of the femoral side (18) of the insert is between 2mm and 6 mm inclusive and is preferably of the order of 4 mm.

23. A knee joint prosthesis as claimed in any of Claims 13 to 22, **characterised in that** said outer surface (13) of the femoral component (2) defines two regions (16, 17) for flexion rotation in a sagittal plane of which a first region (16) has a first radius of curvature (R1) and a second region (17) has a second radius of curvature (R2), the first radius of curvature (R1) being larger than the second radius of curvature (R2), and **in that** the surface of the femoral side (18) of the insert (3) has substantially the same radius of curvature in the sagittal plane as the first radius of curvature (R1) of the femoral component.

24. A knee joint prosthesis as claimed in any of Claims 13 to 23, **characterised in that** said outer surface (13) of the femoral component (2) has a third radius of curvature (R3) for varus/valgus rotation in a coronal plane, and **in that** the surface of the femoral side (18) of the insert (3) has substantially the same radius of curvature in the coronal plane as the third radius of curvature (R3) of the femoral component (2).

25. A knee joint prosthesis as claimed in any of Claims 13 to 24, **characterised in that** it comprises a unicondylar prosthetic device.

## Patentansprüche

1. Ein Tibiaplateau (1) für eine Kniegelenkprothese, beinhaltend
eine Plattform (4), umfassend eine proximale Auflagefläche (5) und eine distale Befestigungsfläche (6); und
einen Kiel (9), der von der distalen Befestigungsfläche (6) vorsteht und der sich, im Einsatz, in einer anterior-posteriore Richtung in Bezug auf die Position des Plateaus (1) erstreckt; wobei sich der Kiel (9) zentral zu dem Tibiaplateau (1) in einer mediallateralen Richtung befindet und im Wesentlichen eben in einer Sagittalebene liegt, mit einer distalen Kante (10), die sich in Bezug auf die proximale Auflagefläche (5) der Plattform (4) schräg erstreckt;
wobei der Kiel (9) eine im Wesentlichen dreieckige Form aufweist, um eine distale Kante (10) mit einem Scheitelpunkt (11) zu definieren, wobei sich der Scheitelpunkt (11) in Richtung eines posterioren Bereichs des Plateaus (1) befindet, und wobei sich das anteriore und posteriore Ende der distalen Kante (10) auf der distalen Befestigungsfläche (6) befinden und von den jeweiligen Kanten der Plattform (4) mit Abstand angeordnet sind.

2. Tibiaplateau (1) gemäß Anspruch 1, **dadurch gekennzeichnet, dass** der Scheitelpunkt (11) des dreieckigen Kiels (9) zwischen 6 mm und 11 mm von der distalen Befestigungsfläche (6) entfernt liegt.

3. Tibiaplateau (1) gemäß den Ansprüchen 1 oder 2, **dadurch gekennzeichnet, dass** ein Winkel zwischen der distalen Kante (10) des Kiels (9) und der proximalen Auflagefläche (5) zwischen einschließlich 10° und 30° und vorzugsweise etwa 17° beträgt.

4. Tibiaplateau (1) gemäß einem der vorhergehenden Ansprüche, **dadurch gekennzeichnet, dass** sich der Kiel (9) in einer Sagittalebene über die halbe Länge der anterior-posteriore Länge des Plateaus (1) erstreckt.

5. Tibiaplateau (1) gemäß einem der vorhergehenden Ansprüche, **dadurch gekennzeichnet, dass** die proximale Auflagefläche (5) glatt und im Allgemeinen eben ist.

6. Tibiaplateau (1) gemäß einem der vorhergehenden Ansprüche, **dadurch gekennzeichnet, dass** die distale Befestigungsfläche (6) der Plattform (4) zur Anbringung an einer Tibia mittels Knochenzement konfiguriert ist.

7. Tibiaplateau (1) gemäß einem der vorhergehenden Ansprüche, **dadurch gekennzeichnet, dass** die distale Befestigungsfläche (6) der Plattform mit einer oder eine Vielzahl von Zementaussparungen (8) versehen ist.

8. Tibiaplateau (1) gemäß Anspruch 7, **dadurch gekennzeichnet, dass** jede der Zementaussparungen (8) in einer anterior-posterioren Richtung ausgerichtet ist.

9. Tibiaplateau (1) gemäß einem der vorhergehenden Ansprüche, **dadurch gekennzeichnet, dass** die distale Befestigungsfläche (6) der Plattform aufgeraut ist.

10. Tibiaplateau (1) gemäß einem der vorhergehenden Ansprüche, **dadurch gekennzeichnet, dass** die Plattform (4) eine Wand (7) definiert, die an einer interkondylären Kante der proximalen Auflagefläche (5) in einer proximalen Richtung vorsteht.

11. Tibiaplateau (1) gemäß Anspruch 10, **dadurch gekennzeichnet, dass** sich die Wand (7), im Einsatz, in einer Sagittalebene proximal erstreckt.

12. Eine Kniegelenkprothese, beinhaltend ein Tibiaplateau (1) gemäß einem der vorhergehenden Ansprüche.

13. Kniegelenkprothese gemäß Anspruch 12, die zusätzlich Folgendes beinhaltet:
eine femorale Komponente (2), die zur Anbringung an einem Femur angepasst ist, und
einen schwebenden Einsatz (3), der zur Einpassung zwischen der femoralen Komponente (2) und dem Tibiaplateau (1), wenn die Prothese in einen Patienten implantiert wird, angepasst ist, wobei der Einsatz (3) eine femorale Seite (18), die derart geformt ist, um sich während der Beugung des Knies gleitend mit einer Außenfläche (13) der femoralen Komponente (2) zu verbinden, und eine tibiale Seite (19), die derart geformt ist, um sich gleitend mit der proximalen Auflagefläche (5) des Tibiaplateaus (1) zu verbinden, aufweist.

14. Kniegelenkprothese gemäß Anspruch 13, **dadurch gekennzeichnet, dass** der Einsatz (3) um eine Sagittalebene nicht symmetrisch ist.

15. Kniegelenkprothese gemäß Anspruch 14, **dadurch gekennzeichnet, dass** der Einsatz (3) eine interkondyläre Seite (22) aufweist, die in der Länge länger als eine entgegengesetzte Oberflächenseite (23) ist.

16. Kniegelenkprothese gemäß Anspruch 14 oder 15, **dadurch gekennzeichnet, dass** eine Ecke (24) an einem posterioren Ende der interkondylären Seite (22) des Einsatzes (3) verlängert ist, damit sie nach hinten vorsteht.

17. Kniegelenkprothese gemäß einem der Ansprüche 14 bis 16, **dadurch gekennzeichnet, dass** ein Winkel (A1) zwischen der interkondylären Seite (22) und einer posterioren Seite des Einsatzes (3) weniger als 90° beträgt.

18. Kniegelenkprothese gemäß einem der Ansprüche 13 bis 17, **dadurch gekennzeichnet, dass** Ecken zwischen einer Oberflächenseite (23) des Einsatzes (3) und seiner posterioren und anterioren Seite abgerundet sind.

19. Kniegelenkprothese gemäß einem der Ansprüche 13 bis 18, **dadurch gekennzeichnet, dass** eine Oberfläche der Tibiaseite (1) des Einsatzes (3) glatt und im Allgemeinen eben ist.

20. Kniegelenkprothese gemäß einem der Ansprüche 13 bis 19, **dadurch gekennzeichnet, dass** ein anterior-posteriores Profil des Einsatzes (3) im Wesentlichen keilförmig ist, wobei die anteriore Höhe (H2) größer als die posteriore Höhe (H1) ist.

21. Kniegelenkprothese gemäß einem der Ansprüche 13 bis 20, **dadurch gekennzeichnet, dass** in der Sagittalebene der Höhenunterschied zwischen einem tiefsten Punkt der femoralen Seite (18) des Einsatzes (3) und einer posterioren Kante (20) der femoralen Seite (18) des Einsatzes (3) nicht mehr als 3 mm beträgt und vorzugsweise in der Größenordnung von 2 mm liegt.

22. Kniegelenkprothese gemäß einem der Ansprüche 13 bis 21, **dadurch gekennzeichnet, dass** in der Sagittalebene der Höhenunterschied zwischen einem tiefsten Punkt der femoralen Seite (18) des Einsatzes (3) und einer anterioren Kante (21) der femoralen Seite (18) des Einsatzes zwischen einschließlich 2 mm und 6 mm beträgt und vorzugsweise in der Größenordnung von 4 mm liegt.

23. Kniegelenkprothese gemäß einem der Ansprüche 13 bis 22, **dadurch gekennzeichnet, dass** die Außenfläche (13) der femoralen Komponente (2) zwei Bereiche (16, 17) zur Beugungsdrehung in einer Sagittalebene definiert, wobei ein erster Bereich (16) davon einen ersten Krümmungsradius (R1) aufweist und ein zweiter Bereich (17) einen zweiten Krümmungsradius (R2) aufweist, wobei der erste Krümmungsradius (R1) größer als der zweite Krümmungsradius (R2) ist, und dadurch, dass die Fläche der femoralen Seite (18) des Einsatzes (3) im Wesentlichen den gleichen Krümmungsradius in der Sagittalebene wie der erste Krümmungsradius (R1) der femoralen Komponente aufweist.

24. Kniegelenkprothese gemäß einem der Ansprüche 13 bis 23, **dadurch gekennzeichnet, dass** die Außenfläche (13) der femoralen Komponente (2) einen dritten Krümmungsradius (R3) zur Varus-/Valgus-Drehung in einer Koronalebene aufweist, und dadurch, dass die Fläche der femoralen Seite (18) des Einsatzes (3) im Wesentlichen den gleichen Krümmungsradius in der Koronalebene wie der dritte Krümmungsradius (R3) der femoralen Komponente (2) aufweist.

25. Kniegelenkprothese gemäß einem der Ansprüche 13 bis 24, **dadurch gekennzeichnet, dass** sie eine unikondyläre Prothesenvorrichtung beinhaltet.

## Revendications

1. Un plateau tibial (1) pour une prothèse de l'articulation du genou, comprenant
une plate-forme (4) incluant une surface d'appui proximale (5) et une surface de fixation distale (6) ; et
une quille (9) qui fait saillie à partir de la surface de fixation distale (6) et qui s'étend dans une direction antéro-postérieure par rapport à la position du plateau (1) lors de l'utilisation ;
dans lequel la quille (9) se trouve au centre du plateau tibial (1) dans une direction médio-latérale et est substantiellement plane dans un plan sagittal avec un bord distal (10) qui s'étend obliquement par rapport à la surface d'appui proximale (5) de la plate-forme (4) ;
dans lequel la quille (9) est de conformation substantiellement triangulaire pour définir un bord distal (10) ayant un sommet (11), le sommet (11) se trouvant en direction d'un aspect postérieur du plateau (1) et **caractérisé en ce que** les extrémités antérieure et postérieure du bord distal (10) se trouvent sur la surface de fixation distale (6) et sont espacées de bords respectifs de la plate-forme (4).

2. Un plateau tibial (1) tel que revendiqué dans la revendication 1, **caractérisé en ce que** le sommet (11) de la quille triangulaire (9) est à une distance de 6 mm à 11 mm de la surface de fixation distale (6).

3. Un plateau tibial (1) tel que revendiqué dans les revendications 1 ou 2, **caractérisé en ce qu'**un angle entre le bord distal (10) de la quille (9) et la surface d'appui proximale (5) est compris entre 10° et 30° inclus et est de préférence d'approximativement 17°.

4. Un plateau tibial (1) tel que revendiqué dans n'importe quelle revendication précédente, **caractérisé en ce que** la quille (9) s'étend dans un plan sagittal sur la moitié de la longueur de la longueur antéro-postérieure du plateau (1).

5. Un plateau tibial (1) tel que revendiqué dans n'importe quelle revendication précédente, **caractérisé en ce que** la surface d'appui proximale (5) est lisse et généralement plane.

6. Un plateau tibial (1) tel que revendiqué dans n'importe quelle revendication précédente, **caractérisé en ce que** la surface de fixation distale (6) de la plate-forme (4) est configurée pour être attachée à un tibia par un ciment à os.

7. Un plateau tibial (1) tel que revendiqué dans n'importe quelle revendication précédente, **caractérisé en ce que** la surface de fixation distale (6) de la plate-forme est munie d'un évidement ou d'une pluralité d'évidements pour ciment (8).

8. Un plateau tibial (1) tel que revendiqué dans la revendication 7, **caractérisé en ce que** chacun des évidements pour ciment (8) est aligné dans une direction antéro-postérieure.

9. Un plateau tibial (1) tel que revendiqué dans n'importe quelle revendication précédente, **caractérisé en ce que** la surface de fixation distale (6) de la plate-forme est rendue rugueuse.

10. Un plateau tibial (1) tel que revendiqué dans n'importe quelle revendication précédente, **caractérisé en ce que** la plate-forme (4) définit une paroi (7) qui fait saillie dans une direction proximale au niveau d'un bord intercondylien de la surface d'appui proximale (5).

11. Un plateau tibial (1) tel que revendiqué dans la revendication 10, **caractérisé en ce que** la paroi (7) s'étend de façon proximale, lors de l'utilisation, dans un plan sagittal.

12. Une prothèse de l'articulation du genou comprenant un plateau tibial (1) tel que revendiqué dans n'importe quelle revendication précédente.

13. Une prothèse de l'articulation de genou telle que revendiquée dans la revendication 12, qui comprend en plus :
un composant fémoral (2) adapté pour être attaché à un fémur, et
un insert flottant (3) adapté pour tenir entre le composant fémoral (2) et le plateau tibial (1) lorsque la prothèse est implantée chez un patient, l'insert (3) ayant un côté fémoral (18) conformé pour être interconnecté avec une surface externe (13) du composant fémoral (2) de façon à glisser durant la flexion du genou et un côté tibial (19) conformé pour être interconnecté avec la surface d'appui proximale (5) du plateau tibial (1) de façon à glisser.

14. Une prothèse de l'articulation du genou telle que revendiquée dans la revendication 13, **caractérisée en ce que** l'insert (3) n'est pas symétrique autour d'un plan sagittal.

15. Une prothèse de l'articulation du genou telle que revendiquée dans la revendication 14, **caractérisée en ce que** l'insert (3) a un côté intercondylien (22) qui est plus long en longueur qu'un côté superficiel opposé (23).

16. Une prothèse de l'articulation du genou telle que revendiquée dans la revendication 14 ou la revendication 15, **caractérisée en ce qu'**un coin (24) au niveau d'une extrémité postérieure du côté intercondylien (22) de l'insert (3) est étendu de façon à faire saillie vers l'arrière.

17. Une prothèse de l'articulation du genou telle que revendiquée dans n'importe lesquelles des revendications 14 à 16, **caractérisée en ce qu'**un angle (A1) entre le côté intercondylien (22) et un côté postérieur de l'insert (3) est inférieur à 90°.

18. Une prothèse de l'articulation du genou telle que revendiquée dans n'importe lesquelles des revendications 13 à 17, **caractérisée en ce que** des coins entre un côté superficiel (23) de l'insert (3) et ses côtés postérieur et antérieur sont arrondis.

19. Une prothèse de l'articulation du genou telle que revendiquée dans n'importe lesquelles des revendications 13 à 18, **caractérisée en ce qu'**une surface du côté tibial (1) de l'insert (3) est lisse et généralement plane.

20. Une prothèse de l'articulation du genou telle que revendiquée dans n'importe lesquelles des revendications 13 à 19, **caractérisée en ce qu'**un profil antéro-postérieur de l'insert (3) est conformé substantiellement comme une cale, la hauteur antérieure (H2) étant supérieure à la hauteur postérieure (H1).

21. Une prothèse de l'articulation du genou telle que revendiquée dans n'importe lesquelles des revendications 13 à 20, **caractérisée en ce que**, dans le plan sagittal, la différence de hauteur entre un point le plus profond du côté fémoral (18) de l'insert (3) et un bord postérieur (20) du côté fémoral (18) de l'insert (3) ne fait pas plus de 3 mm et est de préférence de l'ordre de 2 mm.

22. Une prothèse de l'articulation du genou telle que revendiquée dans n'importe lesquelles des revendications 13 à 21, **caractérisée en ce que**, dans le plan sagittal, la différence de hauteur entre un point le plus profond du côté fémoral (18) de l'insert (3) et un bord antérieur (21) du côté fémoral (18) de l'insert est compris entre 2 mm et 6 mm inclus et est de préférence de l'ordre de 4 mm.

23. Une prothèse de l'articulation du genou telle que revendiquée dans n'importe lesquelles des revendications 13 à 22, **caractérisée en ce que** ladite surface externe (13) du composant fémoral (2) définit deux régions (16, 17) pour la rotation en flexion dans un plan sagittal, dont une première région (16) ayant un premier rayon de courbure (R1) et une deuxième région (17) ayant un deuxième rayon de courbure (R2), le premier rayon de courbure (R1) étant plus grand que le deuxième rayon de courbure (R2), et **en ce que** la surface du côté fémoral (18) de l'insert (3) a substantiellement le même rayon de courbure dans le plan sagittal que le premier rayon de courbure (R1) du composant fémoral.

24. Une prothèse de l'articulation du genou telle que revendiquée dans n'importe lesquelles des revendications 13 à 23, **caractérisée en ce que** ladite surface externe (13) du composant fémoral (2) a un troisième rayon de courbure (R3) pour la rotation varus/valgus dans un plan frontal, et **en ce que** la surface du côté fémoral (18) de l'insert (3) a substantiellement le même rayon de courbure dans le plan frontal que le troisième rayon de courbure (R3) du composant fémoral (2).

25. Une prothèse de l'articulation du genou telle que revendiquée dans n'importe lesquelles des revendications 13 à 24, **caractérisée en ce qu'**elle comprend un dispositif prothétique unicondylien.
